# EUROPEAN PATENT SPECIFICATION

(11) **EP 0 642 491 B1**
(45) Date of publication and mention of the grant of the patent: **04.08.1999**
(21) Application number: 93910240.6
(22) Date of filing: 20.05.1993
(51) Int. Cl.: C07C 63/26, C07C 51/487, F28C 3/06

(54) **PROCESS FOR THE PRODUCTION OF PURIFIED TEREPHTHALIC ACID**
VERFAHREN ZUR HERSTELLUNG VON GEREINIGTER TERPHTHALSÄURE
PROCEDE DE PRODUCTION D'ACIDE TEREPHTALIQUE PURIFIE

(30) Priority: 29.05.1992 GB 9211415
(43) Date of publication of application: 15.03.1995
(73) Proprietor: E.I. DU PONT DE NEMOURS AND COMPANY, Wilmington Delaware 19898 (US)
(72) Inventor: TURNER, John Arthur, Middlesbrough, Cleveland TS6 0SW (GB); HINDMARSH, Eric, Nr. Whitby, North Yorks YO21 2LE (GB); PARKER, David, Great Ayton, Middlesbrough TS9 6ER (GB); MILNE, Ian, Peter, Stockton-on-Tees TS18 3QH (GB)
(74) Representative: Jones, Alan John
(86) International application number: GB9301033
(87) International publication number: WO9324441

(56) References cited:
- DE-A- 2 601 431
- GB-A- 1 163 665
- US-A- 4 405 809
- US-A- 4 500 732

## Description

This invention relates to a process for the production of purified terephthalic acid.

Purified terephthalic acid (PTA) may be obtained by purification of crude terephthalic acid (CTA) which is conventionally produced by the oxidation of p-xylene in the presence of oxygen and a catalyst in a liquid phase comprising for example acetic acid. CTA typically contains impurities for example catalyst residues and by-products of the oxidation of p-xylene such as benzoic acid, p-toluic acid, trimellitic acid and especially 4-carboxybenzaldehyde (4-CBA). CTA may also be produced by the hydrolysis of polyalkylene terephthalate for example, polyethylene terephthalate.

The purification process conventionally involves mixing the CTA with an aqueous medium which, at low temperatures, provides a slurry of CTA. The slurry of CTA is heated to dissolve the terephthalic acid in the aqueous medium to provide an aqueous terephthalic acid solution which may contain organic impurities for example 4-CBA. This aqueous solution is then contacted at elevated pressure and temperature with hydrogen, preferably in the presence of a catalyst, under reducing conditions to reduce chemically at least a proportion of the impurities. Following hydrogen treatment, the terephthalic solution is subjected to a crystallisation process in which the pressure and temperature of the solution is decreased, resulting in the formation of PTA crystals which are then recovered. As disclosed in for example US Patents Nos. 4500732 and 4405809, conventional practice is to pass water evaporated in the crystallisation process to a recycle solvent drum. It is also known from US Patent No. 3584039 to condense evaporated water produced in the crystallisation process and recycle the condensate for dissolution of CTA.

Energy is required in order to heat the slurry of CTA in aqueous medium to at least a temperature at which the terephthalic acid dissolves in the said medium. Hitherto this energy has been derived at least in part by indirect heat transfer from a material such as steam or water derived from another stage of the terephthalic acid production process by using a series of conventional shell and tube heat exchangers. Provision of such heat exchangers in a terephthalic acid production process incurs significant capital expense. Furthermore, heat is transferred across a surface and inevitably heat loss is encountered in such heat exchangers.

There are further problems associated with using such heat exchangers in a terephthalic acid production process. They may suffer from fouling due to the fact that the process stream is a slurry of a solid in a liquid phase. Furthermore, large quantities of heat transfer fluid are required to provide acceptable heat transfer. German Patent No. 289891 discloses one proposal for overcoming the problem of fouling by employing a series of six heat exchangers in which use is made of steam derived from the crystallisation process to effect heating of the water-terephthalic acid suspension.

The present invention provides means by which these problems may be avoided or reduced.

Accordingly this invention provides a process for the production of purified terephthalic acid (PTA) from crude terephthalic acid (CTA) which contains at least one organic impurity comprising mixing the CTA with an aqueous medium to form an aqueous CTA mixture, heating the mixture to dissolve substantially all of the CTA in the aqueous medium and thereby produce a solution of CTA, hydrogenating the aqueous CTA solution to reduce chemically at least some of the said organic impurity and to form an aqueous stream of purified terephthalic acid (PTA), and decreasing the pressure and temperature of the aqueous PTA stream to effect crystallisation of PTA, said heating of the mixture of CTA with aqueous medium being effected at least in part by introducing into the mixture steam having a temperature greater than that of said aqueous mixture, characterised in that the steam so derived contains paratoluic acid and is introduced into said mixture via at least two steam injection stages each supplied with steam from respective stages of the crystallisation section in such a way that steam from a higher temperature and pressure crystallisation stage is supplied to an injection device downstream of an injection device supplied with steam from a lower temperature and pressure crystallisation stage, the pressure of the CTA mixture being increased as it passes from one steam injection stage to the next.

Preferably the aqueous mixture is caused to flow in the form of a stream and the vapour is introduced into the flowing stream. CTA in aqueous medium, whether it be in the form of a slurry or a solution, is referred to herein as the CTA stream.

By reducing or avoiding fouling in the heating of the CTA stream, the present invention provides significant advantages in that there is more efficient heating of the CTA stream and the maintenance and hence process interruptions are reduced correspondingly.

Use of steam recovered from within the terephthalic acid production process enables recoverable energy to be re-used advantageously in the terephthalic acid production process thus providing a significant cost benefit.

We do not exclude the possibility that the steam itself is generated from the liquid phase, for example as a result of flashing in the course of transferring the liquid phase from a higher pressure to a lower pressure. Also the steam may itself be one component of a two phase mixture comprising the vapour phase and a liquid phase, eg produced as a result of transferring a liquid phase from higher to lower pressure with accompanying flashing of part of the liquid phase to the vapour phase. In this instance, the two phase mixture (vapour and liquid phase components) may be mixed with the CTA stream in order to heat the same or the vapour phase component may first be disengaged from the two phase mixture and then used to heat the CTA stream by mixing of the vapour phase with the CTA stream. Thus, in one embodiment of the invention, steam derived from the crystallisation pressure at a predetermined pressure may be initially condensed and transferred to a mixing zone at a lower pressure with accompanying flashing resulting in a two phase mixture of steam produced by flashing and liquid phase water, which mixture is then added to the CTA stream to heat the latter.

Desirably, the steam is obtained by separation of at least one component other than terephthalic acid from the PTA stream during reduction of the pressure and temperature to effect crystallisation of PTA. The vapour is suitably formed by evaporation of water from the aqueous terephthalic acid stream to produce steam. Suitably, at least part of the separated component is recycled and introduced into the CTA stream thereby to effect heating of the CTA stream. By recycling the separated component into the process, the problems of disposal of the separated component are reduced. The separated component for recycling is referred to herein as the recycle stream.

In addition to heating the CTA stream by introducing steam into it, heating of the stream may also be effected by one or more conventional heat exchangers. In such an arrangement the conventional heat exchangers provide only a fraction of the heating effect, therefore the problems associated with the use of such exchangers are consequently reduced as compared with a process in which the total heating effect is provided conventionally.

Preferably, in terms of the heat energy requirement for dissolution of CTA in said aqueous medium, of the total heat energy input provided by the direct introduction of the steam and by heat exchanger(s), at least 15%, more preferably at least 30% and most preferably at least 50%, is provided by the steam.

The recycle stream may be added to the CTA stream, preferably to the slurry, at the point of or subsequent to that at which the CTA is mixed with the aqueous medium. The recycle stream may be used to pre-heat the CTA slurry thereby reducing the amount of additional energy required to heat it up to the temperature at which the terephthalic acid dissolves in the aqueous medium.

Suitably, the CTA stream is heated to substantially the same temperature as that at which the reduction is carried out.

The reduction stage is suitably carried out by passing the terephthalic acid stream through a bed of catalyst, preferably a flooded bed of heterogeneous catalyst. The heterogeneous catalyst may be a supported noble metal catalyst, for example platinum and/or preferably palladium on an inert, for example carbon, support. The reduction is suitably carried out at a temperature of 250 to 350°C and a hydrogen partial pressure of 10 bara, preferably 1 to 50 and more preferably 5 to 25 bara. The total system pressure is suitably, 50 to 100 bars absolute. The terephthalic acid stream to be passed through the reduction stage suitably comprises 20 to 50% by weight of terephthalic acid.

The pressure of the terepthalic acid stream after reduction is suitably decreased whereby the stream is cooled desirably to a temperature of 100 to 250°C, preferably 100 to 210°C, to provide a slurry of PTA in an aqueous solution.

The pressure is preferably decreased by passing the PTA stream through a series of at least 2 pressure let-down vessels for example crystallisers, in which at least part of the aqueous medium is separated, for example by evaporation, as a recycle stream. The pressure let-down vessels are desirably operated at a pressure and temperature not greater than that of the hydrogenation stage.

Each pressure let-down vessel is desirably operated at a pressure and temperature which is lower than that of the preceding vessel or for the first vessel, not greater than that of the hydrogenation reactor thus providing a stepwise cooling of the PTA stream to produce the solid PTA.

The steam supplied to the downstream injection device is desirably recovered from the first crystallisation vessel into which the PTA stream passes after leaving the hydrogenation stage. Recovery from such a vessel allows recovery of high-grade heat.

Suitably the steam to be injected is at a pressure of at least 1 bara, preferably at least 10 bara, more preferably at least 20 bara and especially at least 40 bara and is at a temperature of at least 100°C, preferably at least 140°C and especially at least 160°C.

The steam may be recycled at any desired rate of flow but the rate may be reduced by splitting the steam supply to provide a stream for recycle to heat the CTA stream and a second stream suitable for use in pre-heating other stages of the terephthalic acid production process or in energy recovery operations.

By recycling the steam into the CTA stream, there may be a build up of certain materials contained in the separated component within the system. Such materials may be removed by a suitable purge mechanism and may be further processed or disposed of as required.

A preferred purge mechanism comprises passing the CTA stream into a vessel in which there is free head-space and removing gaseous materials, for example non-condensibles, in the head-space, from the vessel. Any such purge mechanism should be employed at a point down-stream of the point at which the steam is introduced into the crude terepthalic acid stream.

In one implementation of the present invention there is provided a process for the production of purified terephthalic acid (PTA) comprising:
(a) forming an aqueous slurry of crude terephthalic acid (CTA) containing, inter alia, 4-carboxybenzaldehyde and p-toluic acid, using water of which at least part is recycled and contains p-toluic acid additional to that present in the CTA and heating the slurry to produce an aqueous CTA solution having an equivalent total p-toluic acid content of at least 3000 ppmw relative to the CTA content of the solution;
(b) subjecting the aqueous solution of CTA to hydrogenation to convert, inter alia, at least part of the 4-CBA content of the CTA into p-toluic acid;
(c) effecting controlled stagewise crystallisation of the hydrogenated solution to crystallise PTA;
(d) terminating the crystallisation at an elevated temperature and pressure such that the crystallised TA contains no more than about 150 ppmw p-toluic acid;
(e) subjecting the purified TA to separation and washing processes in which the purified TA is separated from the mother liquor, washed and separated from the wash liquor to derive a mass of PTA crystals, such separation and washing being carried out in a zone at elevated temperature and pressure such that the p-toluic acid content of the separated and washed PTA is maintained at a level no greater than 150 ppmw relative to the PTA;
(f) recycling at least part of the mother liquor separated from the PTA in step (e), together with at least part of its p-toluic acid content, to step (a) whereby the recycled mother liquor constitutes said recycled water used in step (a); and
(g) combining p-toluic acid-containing steam derived from respective stages of step (c) with said slurry of step (a) to effect heating thereof.

In the course of carrying out the purification reaction, para-toluic acid is present and is also generated by the hydrogenation of 4-CBA. Para-toluic acid is substantially more soluble in water than terephthalic acid which means that controlled crystallisation of the solution resulting from the hydrogenation reaction allows terephthalic acid to be precipitated in such a way that only small amounts of para-toluic acid (typically of the order of 150, more 120 ppmw or less based on the amount of solid terephthalic acid) are co-precipitated, the remaining para-toluic acid being left in solution. Conventionally, the level of para-toluic acid or precursor thereof (4-CBA) that can be tolerated in the solution to be hydrogenated is limited by the need to secure a purified terephthalic acid product with no more than a predetermined para-toluic acid content, typically no greater than 150 ppmw, and more preferably 120 ppmw.

In conventional terephthalic acid production, the para-toluic acid present in the aqueous CTA solution following the purification reaction derives from two sources. One such source is para-toluic acid produced in the course of the liquid phase oxidation of para-xylene. Another source of para-toluic acid is 4-CBA which results from the incomplete oxidation of para-xylene and a substantial part of which, when subjected to hydrogenation in the course of purification of crude terephthalic acid, is converted to para-toluic acid. Thus, the aqueous CTA solution supplied to the purification reaction may be considered to contain an "equivalent total p-toluic acid content", namely the p-toluic acid actually present and that derivable from a precursor, eg 4-CBA. In conventional terephthalic acid production, one would not contemplate introducing unnecessary para-toluic acid into the process, eg by using steam derived from the crystallisation process which will inevitably contain p-toluic acid.

In the purification stage as operated conventionally, a relatively large quantity of demineralised water is employed, especially as solvent for the crude terephthalic acid and as a wash liquid for the purified terephthalic acid. Such water becomes contaminated and may present problems of disposal and, if discarded, may involve loss of potentially useful materials, such as terephthalic acid and/or its precursors.

Our prior EP-A-498591 discloses a process for the production of terephthalic acid by the liquid phase oxidation of para-xylene in which mother liquor (comprising mainly water) obtained following conventional crystallisation of purified terephthalic acid in the purification stage is treated to recover further terephthalic acid (and co-precipitated p-toluic acid). The remaining mother liquor comprises mainly water and is at least in part recycled for the purpose of dissolving crude terephthalic acid prior to hydrogenation thereof. The remaining mother liquor or part thereof may be cooled further to a temperature in the range 15°C to 100°C or separated for example by filtration, to produce a less pure precipitate of terephthalic acid and a secondary mother liquor. Mother liquor from this separation and/or the less pure precipitate may also be recycled as desired, as disclosed in EP-A-498591.

Recycling of the mother liquor inevitably re-introduces p-toluic acid into the process. The additional p-toluic acid can however be tolerated whilst still achieving a level of no more than 150 ppmw (more preferably no more than 120 ppmw) p-toluic acid impurity in the purified terephthalic acid, by tailoring the crystallisation process in such a way that the various stages of pressure let-down result in terephthalic acid of the desired purity. In particular, the crystallisation process is tailored so as to limit the amount of p-toluic acid co-precipitating with terephthalic acid and an important factor in this respect is setting the temperature of the final crystallisation stage at a level where p-toluic acid is still relatively soluble in the aqueous mother liquor. A suitable temperature for this purpose will be at least 160°C, eg 170°C or greater.

By operating the terephthalic acid production process using recycled aqueous mother liquor containing p-toluic acid to effect slurrying of CTA prior to the hydrogenation process and by tailoring the crystallisation process to limit the p-toluic acid content of the purified terephthalic acid to 150 ppmw or less (relative to the solid purified terephthalic acid) then the use of steam derived from the crystallisation process for heating the CTA slurry can be more readily tolerated even though the steam so obtained will inevitably contain p-toluic acid. If the process is operated in a regime in which mother liquor containing p-toluic acid is recycled and combined with CTA, the amount of p-toluic acid re-introduced by means of the steam recovered from the crystallisation process represents a relatively small proportion of the equivalent total p-toluic acid entering purification process; for instance, the equivalent total p-toluic acid content of the aqueous CTA solution prior to hydrogenation may contain at least 3000 ppmw p-toluic acid equivalent, for instance at least 3500 ppmw and up to for example 7000 ppmw. In contrast, if steam derived from the crystallisation process in a conventional PTA production process is used directly to heat the CTA slurry, the p-toluic acid re-introduced in these circumstances would constitute a significantly larger proportion of the equivalent total p-toluic acid entering the purification process.

Preferably in step (e) of said further aspect of the invention, the separation and washing processes are integrated, ie. carried out within the same item of equipment without an intermediate slurrying of the terephthalic acid in water prior to the washing step.

Advantageously, the integrated separation process of step (e) is effected under elevated pressure conditions using a respective belt filter system such as a Pannevis filter. The CTA is typically obtained by the oxidation of p-xylene in the presence of a gas comprising oxygen, for example air, and a bromine-containing catalyst, preferably comprising a heavy metal for example, manganese or cobalt, in a liquid reaction phase comprising an organic carboxylic acid. The organic carboxylic acid preferably contains 2 to 6 carbon atoms. Acetic acid is particularly suitable.

Where the CTA is produced as a slurry in a reaction medium, substantially all the CTA may be separated from the reaction medium for example by a rotary vacuum filter, dried and then mixed with an aqueous medium to produce the terephthalic acid stream. However, it is preferred that the terephthalic acid stream is produced by displacing the reaction medium by an aqueous medium in a continuous counter current washing and filtration process as disclosed in our prior EP-A-502628.

Suitably the oxidation is carried out at a pressure of 5 to 30 bars absolute, and preferably an oxygen concentration of 0 to 8% in the gas leaving the reactor and at a temperature of 150 to 250°C. It is suitably a continuous process, and is preferably carried out in a stirred reactor.

The oxidation reaction is exothermic and the heat of the reaction may conveniently be removed by separation of water and carboxylic acid from the reaction medium. This also provides a means by which the water of reaction is removed. The stream of carboxylic acid and water may also comprise other gases such as oxygen. This stream is preferably distilled to remove water therefrom to provide a carboxylic acid of lower water content which is desirably recycled to the oxidation reactor.

Instead of production by the liquid phase oxidation of para-xylene, the CTA may be derived by treatment of a polyalkylene terephthalate, for instance by means of a process such as that disclosed in our prior European Patent Application No. 92311421.9.

The purification process may be conducted in two stages, namely a first stage in which at least part of the 4-CBA content of the CTA is oxidised to terephthalic acid by means of a suitable oxidising agent (which may be in the gaseous phase, eg oxygen gas, or the liquid phase), and a second stage in which the CTA is subjected to the aforesaid hydrogenation treatment (which serves to convert impurities in the form of so-called colour bodies to forms which can be tolerated in the end product). Where such a two-stage process is employed, the CTA will usually be dissolved to form an aqueous solution prior to the first stage, and heating of the aqueous medium to aid dissolution will be effected by introduction of hot vapour into the CTA mixture.

The steam may be injected into the CTA stream by any suitable means. A particularly suitable means and a further aspect of the invention comprises an inlet apparatus for effecting intimate mixing of a process stream with a fluid which comprises a longitudinally extending hollow member through which the process stream may pass, the said apparatus having inlet means in communication with the interior of the hollow member, for receiving the fluid and a screen disposed within the said member which is permeable to the fluid such that the fluid passes through the screen thereby to contact the process stream and be mixed intimately therewith.

Conveniently the screen extends longitudinally within the hollow member.

This apparatus provides for efficient contact between the process stream and the fluid as the screen serves to interrupt the flow of the fluid and effectively provides a large number of small flows of the fluid into the process stream. It is particularly beneficial for mixing a process stream comprising terephthalic acid in an aqueous medium and a fluid comprising steam.

Desirably the hollow member is substantially cylindrical in cross section along at least a part, and preferably all, of its length thus facilitating incorporation in pipelines, the majority of which are cylindrical, in for example chemical plant.

Desirably the screen is arranged concentrically within the hollow member. Suitably the screen is a hollow body having a plurality of orifices. The hollow body is desirably of a similar shape but reduced cross section to the hollow member and preferably comprises a cylindrical member.

Suitably the orifices are disposed in the hollow body in a regular array. In cases where the hollow body is cylindrical, the orifices are preferably arranged in a succession of circumferentially disposed rings of orifices, the orifices in each ring being regularly spaced around the cylindrical body and the rings being regularly spaced along the length of the said body. Desirably, the orifices in any particular ring are offset in a circumferential direction from those in an adjacent ring of orifices.

The orifices in the body are preferably disposed at such an angle in the body that, in use, the angle (α) between the direction of flow of the fluid and that of the process stream is not greater than 90° and preferably not greater than 75°.

The screen whilst being permeable to the fluid is desirably arranged within the hollow member such that at most, only a minor proportion of the process stream contacts the fluid prior to the fluid passing through the screen.

This apparatus may be employed as an insert into conventional pipelines especially in chemical plant and is suitably of such shape and dimensions that the flow of the process stream is substantially uninterrupted by it.

The invention will now be described by way of example only with reference to the accompanying drawings, in which:
Figure 1 is a flow diagram showing application of the present invention to one form of plant for the production of terephthalic acid;
Figure 2 is a schematic view in longitudinal section of a device for injecting steam into a slurry of CTA in water; and
Figure 3 is a diagrammatic view illustrating the use of steam injection at a number of points into a flow of aqueous CTA slurry.

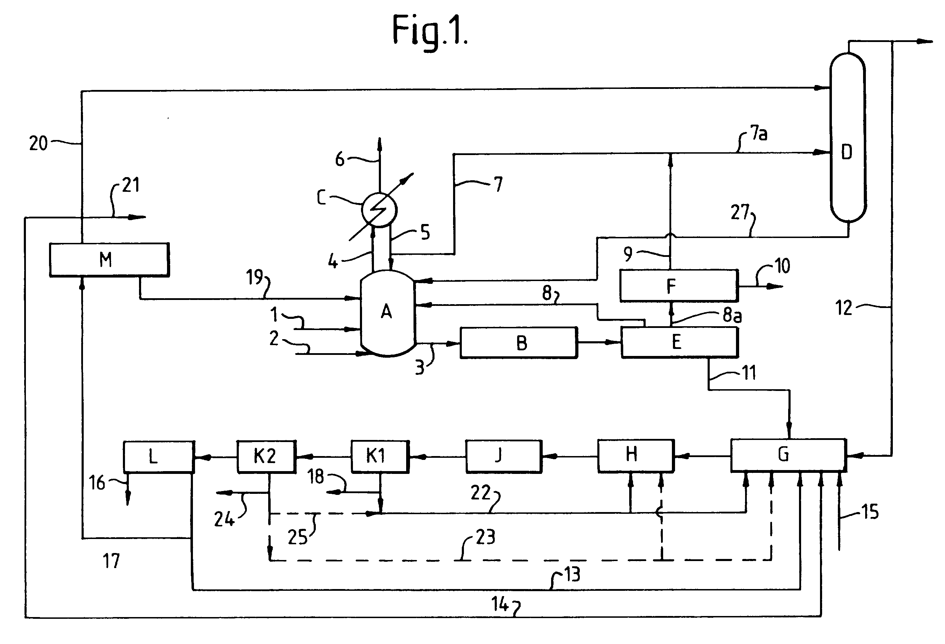

In Fig 1, reactor A is fed with paraxylene and acetic acid containing a dissolved catalyst comprising cobalt, manganese and bromine ions by line 1 and with air via line 2. Reactor A is operated at elevated temperature and pressure. Product from the reactor A is passed to crystallisation stage B by line 3. The temperature within the reactor A is regulated by withdrawing a mixture of acetic acid and water vapour from the reactor and passing the mixture to a condensing system C via line 4. All or most of the condensate is returned to the reactor via line 5 with noncondensibles venting via line 6. To control the water content of the reactor A, part of the condensate is removed from the condensing system C via lines 7, 7a and passed to a distillation column D.

In the crystallisation stage B the temperature is decreased to approximately 75°C to 120°C and the slurry containing crystalline CTA in mother liquor thereby produced is passed to filtration stage E, in which integrated separation and washing processes may be carried out as described in our prior EP-A-502628. Mother liquor recovered from stage E is returned in part via line 8 to the reactor A usually by first mixing with the fresh catalyst, paraxylene and acetic acid contained in line 1. The remaining mother liquor is passed via line 8a to an evaporation stage F in which acetic acid is recovered and supplied by lines 9, 7a to the distillation column D. A purge of by-products and other materials is withdrawn via stream 10. Acetic acid recovered from the distillation column may be returned at least in part to the reactor A via line 27.

From the filtration stage E, solid CTA crystals are passed via stream 11 to a reslurry vessel G where the crystals are reslurried with water recovered from the distillation column D via stream 12 and other water which may be recycled mother liquor via stream 13, recycle mother liquor via stream 14, demineralised water via stream 15 and steam from the crystallisation section K1, K2 as described hereinafter.

5 The suspension of CTA in water produced in stage G is heated in section G and/or H to a temperature of for example 250°C to 350°C to form a solution using steam derived from the crystallisation section K1, K2. The solution is passed to reactor J in which it is reacted with hydrogen over a fixed bed palladium catalyst at elevated pressure thus reducing impurities and/or converting undesirable impurities such as so-called colour bodies to forms which can be tolerated in the end product. The solution is then passed to crystallisation section K1, K2 in which PTA is crystallised.

Within the crystallisation section, the pressure and temperature of the solution is decreased with consequent evaporation of steam which is suitably passed back into reslurry vessel G into the slurry of CTA in water and/or section H as described hereinafter and may in part be purged or passed to other stages of the PTA production process via lines 18 and 24. The temperature to which the solution is cooled in the crystallisation section K1, K2 and the rapidity of cooling is adjusted to produce the appropriate purity of the desired terephthalic acid product.

PTA is separated, washed and dried in stage stage L which is suitably a centrifuge or filter and rotary drier or fluidised bed drier. The stage L conveniently includes an integrated separation and washing stage in the form of a continuous band or belt filter unit such as a Pannevis filter of the form generally described in Filtration and Separation (Page 176 et seq, March/April 1979) and as described in our prior EP-A-502628. The PTA product is recovered from stage L via line 16 and the mother liquor from the separation is passed via line 17 to recovery stage M in which the liquor is evaporated or preferably further cooled so as to permit the recovery of further solids which may be passed back to reactor A via stream 19. The mother liquor recovered from stage M may at least in part be passed back to the distillation column D via line 20, in part be returned to the reslurry section G via stream 14 and in part be purged via stream 21. Preferably if evaporation is used, the evaporated water is returned to the reslurry stage G to be mixed with CTA.

The illustrated embodiment is shown as comprising two crystallisation stages K1 and K2 but it will be appreciated that there may be more. Crystallisation stage K₁ is operated at a higher pressure and temperature than crystallisation stage K₂. The steam from K₁ may be passed back to reslurry vessel G and/or section H separately via lines 22 and 23 respectively without there being any mixing of the steam from K₁ and K₂ or may in part be purged or passed to other stages of the PTA production process via lines 18 and 24 respectively. As the steam from K₁ is at higher pressure and temperature than that from K₂, it is preferably passed back into vessel G or section H at a point downstream of the point at which steam from crystallisation stage K₂ is passed back.

Any steam which is purged or used elsewhere via lines 18 and 24 may be combined into a single stream if desired.

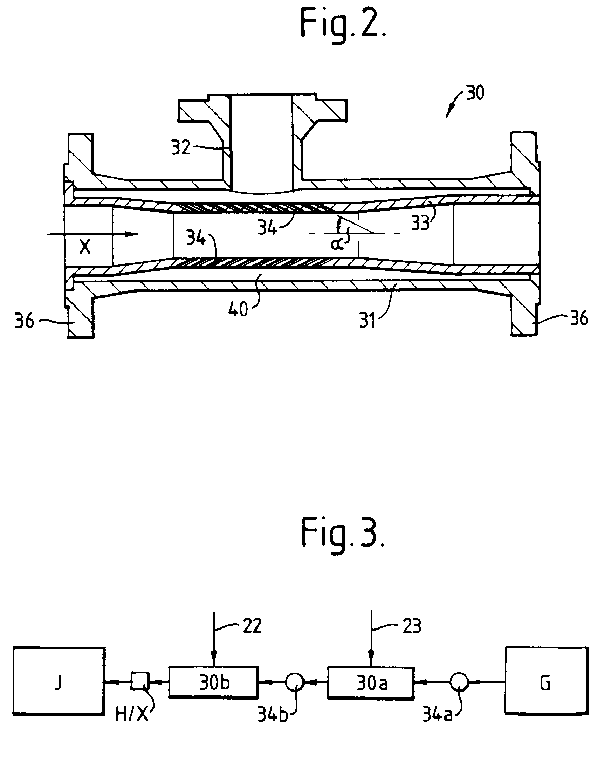

In contrast with conventional practice in which the suspension of CTA and water produced in reslurry vessel is heated indirectly by heat exchange with a heating fluid such as steam, the present invention effects heating of the suspension by injecting the heating medium directly into the suspension and in a preferred feature of the invention the heating medium is constituted by steam derived directly from the crystallisation section K1, K2 (ie without any intermediate condensation and reheating). Injection of the heating medium can be effected efficiently by means of the device shown in Figure 2 at any suitable locations in sections G and H.

As shown, the device 30 comprises a tubular housing 31 of cylindrical section within which is mounted an axial tubular duct 33 also of cylindrical section and through which an axially directed process stream comprising a suspension of CTA in water flows in the direction of arrow X. The housing 31 has an inlet 32 disposed transversely of the axis of the housing and through which steam to be injected into the CTA/water stream is introduced. The tubular duct 33 is mounted within the housing 31 in concentric relation therewith and is of smaller diameter so as to define an annular chamber 40. The duct 33 is in the form of a Venturi and the throat of the Venturi is perforated with an array of orifices 34 which are distributed circumferentially and axially of the duct. Typically, the size of orifices 34 is 3 to 8 mm, preferably 4 to 6 mm. The steam entering the chamber 40 is thereby distributed over the duct 33 and penetrates into the interior of the duct via the orifices 34. On entry into the duct interior, the steam penetrates, and mixes intimately with, the process stream to effect efficient transfer of heat to the CTA/water suspension. Experimental trials indicate that steam injection velocities in range of 30 to 200 mlsec are usually acceptable, with a preference for steam injection velocities between 50 and 150 m/sec. Pressure differentials between the process stream and the injected steam may be as low as 0.3 bar but are preferably 0.5 bar or greater. The orifices 34 are oriented with their axes extending obliquely (at an angle α) to the direction X of the flow of the CTA/water stream through the duct 33. The angle α is usually no greater than 75°. The number of orifices 34 is governed by mass balance considerations. The housing 31 is provided at each end with flanges 36 to enable the device to be coupled into a pipeline for transport of the CTA slurry.

The Venturi section serves to provide a local reduction in pressure of the CTA slurry in the vicinity of steam introduction with an accompanying increase in the slurry velocity. The steam pressure must be in excess of the slurry pressure. Usually the steam will be derived from sources which produce steam under sufficient pressure for this purpose but, if the steam pressure is inadequate, the steam may be pre-compressed prior to introduction into the device 30.

Referring to Figure 3, one arrangement for effecting steam injection is illustrated. As shown, following mixing of CTA and aqueous medium (eg. water with some impurities such as paratoluic acid present) in the reslurry vessel G, the mixture is caused to flow as a stream along a pipeline through a number of steam injection devices 30a, 30b, each as shown in Figure 2, and a heat exchanger HIX prior to feed of the resulting hot aqueous solution of CTA to the reactor J. Thus, the heat input to the CTA stream in order to effect dissolution of the CTA is provided by a combination of conventional heat exchange by means of heat exchanger H/X and by direct injection of steam by means of devices 30a, 30b. In terms of the particular process illustrated in Figure 1, the steam injected by the upstream device 30a is derived from the lower pressure and temperature crystalliser stage K2 via line 23 whilst that injected by device 30b is derived from the higher pressure and temperature stage K1 via line 22.

To maximise the outlet temperature at each injection stage, each injection stage is conveniently preceded by a pumping stage 34a and 34b to increase the pressure of the CTA slurry so as to raise its boiling temperature and thereby prevent bubbling within the CTA stream while allowing for sufficient pressure differential between the pressurised CTA stream and the heating vapour introduced via the injector to permit introduction of the latter into the CTA stream.

In an experimental trial using a device as shown in Figure 3 in order to heat a process stream having a slurry strength of 29% w/w terephthalic acid at a temperature of 136°C, steam at 18 bara and 240°C was injected into the slurry in an amount of 0.074 tonne steam per tonne of slurry feed. The heated slurry exiting the device was found to have a temperature of 180°C, which is in agreement with the heat balance, ie indicating a rapid condensation of the steam to achieve equilibrium.

## Claims

1. A process for the production of purified terephthalic acid (PTA) from crude terephthalic acid (CTA) which contains at least one organic impurity comprising mixing the CTA with an aqueous medium to form an aqueous CTA mixture, heating the mixture to dissolve substantially all of the CTA in the aqueous medium and thereby produce a solution of CTA, hydrogenating the aqueous CTA solution to reduce chemically at least some of the said organic impurity and to form an aqueous stream of purified terephthalic acid (PTA), and decreasing the pressure and temperature of the aqueous PTA stream to effect crystallisation of PTA, said heating of the mixture of CTA with aqueous medium being effected at least in part by introducing into the mixture steam having a temperature greater than that of said aqueous mixture, the steam being derived from the crystallisation section, characterised in that the steam so derived contains paratoluic acid and is introduced into said mixture via at least two steam injection stages each supplied with steam from respective stages of the crystallisation section in such a way that steam from a higher temperature and pressure crystallisation stage is supplied to an injection device downstream of an injection device supplied with steam from a lower temperature and pressure crystallisation stage, the pressure of the CTA mixture being increased as it passes from one steam injection stage to the next.

2. A process as claimed in Claim 1 in which heating of the CTA mixture is effected by introducing said steam into the mixture and additionally by heat transfer between the mixture and a heating fluid within a heat exchanger.

3. A process as claimed in Claim 1 in which the steam is injected into the aqueous mixture as it flows through a conduit.

4. A process as claimed in Claim 1 in which the pressure of the flowing stream of aqueous mixture is locally reduced in each steam injection stage by passage through the throat of a Venturi section and steam is injected into the aqueous mixture while the latter is under reduced pressure conditions.

5. A process as claimed in any one of Claims 1 to 4 in which:
(a) the CTA contains, inter alia, 4-carboxybenzaldehyde and p-toluic acid,
(b) in the production of the aqueous mixture, the CTA is mixed with water of which at least part is recycled and contains p-toluic acid additional to that present in the CTA to produce a slurry which is heated to produce an aqueous CTA solution having an equivalent total p-toluic acid content of at least 3000 ppmw relative to the CTA content of the solution,
(c) subjecting the aqueous CTA solution to hydrogenation,
(d) crystallising the hydrogenated solution and terminating the crystallisation process at an elevated temperature and pressure such that the purified and crystallised (PTA) contains no more than about 150 ppmw p-toluic acid,
(e) the PTA crystals are subjected to separation and washing processes in which the PTA crystals are separated from the mother liquor, washed and separated from the wash liquor to derive a mass of PTA crystals, such separation and washing being carried out in a zone at elevated temperature and pressure such that the p-toluic acid content of the separated and washed PTA is maintained at a level no greater than 150 ppmw relative to the PTA,
(f) at least part of the mother liquor separated from the PTA crystals, together with at least part of its p-toluic acid content, is recycled to step (b) whereby the recycled mother liquor constitutes said recycled water used in step (b); and
(g) combining p-toluic acid-containing steam derived from step (d) with said slurry of step (b) to effect heating thereof in accordance with Claim 1.

## Patentansprüche

1. Verfahren zur Herstellung von gereinigter Terephthalsäure (PTA) aus roher Terephthalsäure (CTA), die wenigstens eine organische Verunreinigung enthält, Verfahren das die folgenden Schritte aufweist: ein Mischen der CTA mit einem wäßrigen Medium um eine wäßrige CTA-Mischung zu bilden, ein Erwärmen der Mischung um im wesentlichen alle CTA in dem wäßrigen Medium zu lösen und auf diese Weise eine Lösung von CTA herzustellen, eine Hydrierung der wäßrigen CTA-Lösung um wenigstens einen Teil der organischen Verunreinigung auf dem chemischen Wege zu reduzieren und um einen wäßrigen Strom von gereinigter Terephthalsäure (PTA) zu bilden, sowie ein Verringern des Druckes und der Temperatur des wäßrigen PTA-Stroms um eine Kristallisation des PTA zu bewirken, wobei das Erwärmen der Mischung der CTA mit dem wäßrigen Medium wenigstens zum Teil bewerkstelligt wird durch ein Einführen in die Mischung von Dampf der eine höhere Temperatur aufweist als diejenige der besagten wäßrigen Mischung, während der Dampf aus der Kristallisationsstufe abgeleitet wird, dadurch gekennzeichnet, daß der so abgeleitete Dampf para-Toluylsäure enthält und mit der Hilfe von wenigstens zwei Dampfeinspritzstufen in die Mischung eingeführt wird, wobei eine jede mit Dampf aus den jeweiligen Stufen der Kristallisationsstufe versorgt wird, auf solche Weise, daß Dampf aus einer Kristallisationsstufe mit einer höheren Temperatur und einem höheren Druck einer Einspritzvorrichtung zugeführt wird, welche stromabwärts gelegenen ist in Bezug auf eine Einspritzvorrichtung, die mit Dampf von einer Kristallisationsstufe mit einer niedrigeren Temperatur und einem niedrigeren Druck versorgt wird, wobei der Druck der CTA-Mischung erhöht wird während sie von der einen Dampfeinspritzstufe zu der nächsten übergeleitet wird.

2. Verfahren gemäß Anspruch 1, bei welchem das Erwärmen der CTA-Mischung durch Einführen des Dampfes in die Mischung und zusätzlich durch eine Wärmeübertragung zwischen der Mischung und einer Heizflüssigkeit mittels eines Wärmeaustauschers bewirkt wird.

3. Verfahren gemäß Anspruch 1, bei welchem der Dampf in die wäßrige Mischung eingespritzt wird, während sie durch eine Leitung strömt.

4. Verfahren gemäß Anspruch 1, bei welchem der Druck des fließenden Stroms der wäßrigen Mischung örtlich in jeder Dampfeinspritzstufe dadurch vermindert wird, daß er durch den Hals eines Venturiprofils hindurch geschickt wird, und Dampf in die wäßrige Mischung eingespritzt wird, während letztere sich unter verminderten Druckbedingungen befindet.

5. Verfahren gemäß irgendeinem der Ansprüche 1 bis 4, bei welchem:
(a) die CTA unter anderem 4-Carboxybenzaldehyd und p-Toluylsäure enthält,
(b) bei der Herstellung der wäßrigen Mischung, die CTA mit Wasser vermischt wird von dem wenigstens ein Teil zurückgeführt worden ist und p-Toluylsäure zusätzlich zu der schon in der CTA vorhandenen enthält, um einen Schlamm herzustellen, welcher erwärmt wird, um eine wäßrige CTA-Lösung mit einer äquivalenten Gesamtmenge an p-Toluylsäure von wenigstens 3000 ppmw in Bezug auf den CTA-Gehalt in der Lösung herzustellen,
(c) die wäßrige CTA-Lösung einer Hydrierung unterzogen wird,
(d) die hydrierte Lösung kristallisiert wird und der Kristallisationsvorgang bei einer erhöhten Temperatur und einem erhöhten Druck zum Abschluß gebracht wird, derart daß das gereinigte und kristallisierte PTA nicht mehr als ungefähr 150 ppmw an p-Toluylsäure enthält,
(e) die PTA-Kristalle einem Abtrennungs- und Waschverfahren unterzogen werden, in welchem die PTA-Kristalle von der Mutterlauge abgetrennt werden, gewaschen und von der Waschflüssigkeit abgetrennt werden, um eine Masse an PTA-Kristallen zu gewinnen, wobei eine solche Abtrennung und ein solches Waschen in einer Zone unter erhöhter Temperatur und erhöhtem Druck durchgeführt werden, derart daß der Gehalt an p-Toluylsäure der abgetrennten und gewaschenen PTA auf einem Niveau gehalten wird der nicht größer als 150 ppmw in Bezug auf die PTA ist,
(f) wenigstens ein Teil der von den PTA-Kristallen abgetrennten Mutterlauge, zusammen mit wenigstens einem Teil ihres Gehaltes an p-Toluylsäure, zu dem Schritt (b) zurückgeführt wird, wodurch die zurückgeführte Mutterlauge das in dem Schritt (b) verwendete zurückgeführte Wasser darstellt; und
(g) der p-Toluylsäure enthaltende Dampf, der von dem Schritt (d) abgeleitet worden ist, mit dem Schlamm aus Schritt (b) kombiniert wird, um ein Erwärmen desselben in Übereinstimmung mit Anspruch 1 zu bewirken.

## Revendications

1. Procédé de production d'acide téréphtalique purifié (PTA) à partir d'un acide téréphtalique brut (CTA), qui contient au moins une impureté organique, comprenant la réalisation d'un mélange du CTA avec un milieu aqueux pour former un mélange aqueux de CTA, le chauffage du mélange pour dissoudre substantiellement la totalité du CTA dans le milieu aqueux et pour produire ainsi une solution de CTA, l'hydrogénation de la solution aqueuse de CTA pour réduire chimiquement au moins une partie de ladite impureté organique et pour former un courant aqueux d'acide téréphtalique purifié (PTA), ainsi que la diminution de la pression et de la température du courant aqueux de PTA pour effectuer la cristallisation du PTA, ledit chauffage du mélange de CTA avec un milieu aqueux étant effectué au moins en partie par l'introduction dans le mélange de vapeur avec une température supérieure à celle dudit mélange aqueux, la vapeur étant dérivée de la section de cristallisation, procédé caractérisé en ce que la vapeur dérivée de cette façon contient de l'acide paratoluique et est introduite dans ledit mélange par l'intermédiaire d'au moins deux étapes d'injection de vapeur, chacune d'elle étant alimentée avec de la vapeur à partir des étapes respectives de la section de cristallisation, de telle manière que de la vapeur venant d'une étape de cristallisation à température et à pression plus élevées est alimentée vers un dispositif d'injection en aval d'un dispositif d'injection alimenté avec de la vapeur venant d'une étape de cristallisation à température et à pression plus basses, la pression du mélange de CTA étant augmentée au moment où il passe d'une étape d'injection de vapeur à la suivante.

2. Procédé suivant la revendication 1, dans lequel le chauffage du mélange de CTA est effectué par l'introduction de ladite vapeur dans le mélange et en plus par un transfert de chaleur entre le mélange et un fluide chauffant dans un échangeur de chaleur.

3. Procédé suivant la revendication 1, dans lequel la vapeur est injectée dans le mélange aqueux au moment où il s'écoule à travers un conduit.

4. Procédé suivant la revendication 1, dans lequel la pression du courant en écoulement du mélange aqueux est réduite localement dans chaque étape d'injection de vapeur par le passage à travers l'étranglement d'une section de Venturi et de la vapeur est injectée dans le mélange aqueux, tandis que ce dernier se trouve sous des conditions de pression réduite.

5. Procédé suivant l'une quelconque des revendications 1 à 4, dans lequel:
(a) le CTA contient, entre autres, du 4-carboxybenzaldéhyde et de l'acide p-toluique,
(b) au cours de la production du mélange aqueux, le CTA est mélangé avec de l'eau dont au moins une partie est recyclée et contient de l'acide p-toluique en plus de celui qui est présent dans le CTA pour produire une suspension épaisse qui est chauffée pour produire une solution aqueuse de CTA avec une teneur équivalente totale en acide p-toluique d'au moins 3000 ppm en poids par rapport à la teneur en CTA de la solution,
(c) la solution aqueuse de CTA est exposée à une hydrogénation,
(d) la solution hydrogénée est cristallisée et le procédé de cristallisation est achevé à une température et à une pression élevées, de sorte que le PTA purifié et cristallisé ne contient pas plus qu'environ 150 ppm en poids d'acide p-toluique,
(e) les cristaux de PTA sont soumis à des procédés de séparation et de lavage dans lesquels les cristaux de PTA sont séparés de la liqueur mère, lavés et séparés de la liqueur de lavage pour dériver une masse de cristaux de PTA, cette séparation et ce lavage étant réalisés dans une zone à température et à pression élevées, de sorte que la teneur en acide p-toluique du PTA séparé et lavé est maintenue à un niveau ne dépassant pas 150 ppm en poids par rapport au PTA,
(f) au moins une partie de la liqueur mère séparée des cristaux de PTA, accompagnée d'au moins une partie de sa teneur en acide p-toluique, est recyclée vers l'étape (b), en conséquence de quoi la liqueur mère recyclée constitue ladite eau recyclée utilisée dans l'étape (b); et
(g) la combinaison de vapeur contenant de l'acide p-toluique dérivée de l'étape (d) avec ladite suspension épaisse de l'étape (b) pour effectuer le chauffage de cette dernière conformément à la revendication 1.
